# EUROPEAN PATENT APPLICATION

(11) **EP 4 484 421 A1**
(43) Date of publication of application: **01.01.2025**
(21) Application number: 23775112.8
(22) Date of filing: 24.03.2023
(51) Int. Cl.: C07D 407/04, C08F 4/40, C08F 2/44, C07D 307/62, A61K 6/30, A61K 6/60, A61K 6/62

(54) **ASCORBIC ACID DERIVATIVE OR SALT THEREOF, ADDITIVE FOR INITIATING POLYMERIZATION, POLYMERIZATION INITIATOR, KIT FOR PREPARING CURABLE COMPOSITION, CURABLE COMPOSITION, CURED PRODUCT AND DENTAL MATERIAL**

(30) Priority: 25.03.2022 JP 2022050804
(71) Applicant: Mitsui Chemicals, Inc., Tokyo 104-0028 (JP)
(72) Inventor: TAKAHASHI, Issei, Sodegaura-shi, Chiba 299-0265 (JP); DANJO, Takahiro, Sodegaura-shi, Chiba 299-0265 (JP)
(74) Representative: Hoffmann Eitle
(86) International application number: PCT/JP2023/012013
(87) International publication number: WO 2023/182519

(57) **Abstract**

An ascorbic acid derivative or a salt thereof, includes a structure in which at least one of hydroxyl groups included in an ascorbic acid is substituted by an ether bond bonded to a carbon atom.

## Description

### Technical Field

The present disclosure relates to an ascorbic acid derivative or a salt thereof, an additive for polymerization initiation, a polymerization initiator, a curable composition preparation kit, a curable composition, a cured product, and a dental material.

### Background Technology

In the dental field, a synthetic resin molding product or the like is used to repair a tooth defect. For example, a curable composition called cement is used as a tooth substitute for repairing a large tooth defect. In recent years, the scope of use of cement has expanded.

A photopolymerization initiator, a chemical polymerization initiator, or the like may be used for polymerization of a curable composition for dental materials such as cement.

For example, one of the common chemical polymerization initiator types is a redox type polymerization initiator that combines an oxidizing agent and a reducing agent. As a redox type polymerization initiator, for example, a polymerization initiator type using an organic peroxide as an oxidizing agent and an aromatic amine compound as a reducing agent is known.

Patent Document 1 discloses a redox type initiator containing ascorbic acid or an ascorbic acid ester, a transition metal component, an organic peroxide, and the like.
[Patent Document 1] International Publication No. 2016/007453

### SUMMARY OF INVENTION

### Technical Problem

However, a cured product obtained by using ascorbic acid or an ascorbic acid ester described in Patent Document 1 has room for improvement in terms of adhesive properties.

The problem to be solved by a one embodiment of the present disclosure is to provide an ascorbic acid derivative or a salt thereof than can improve adhesive properties of an obtained cured product, and an additive for polymerization initiation, a polymerization initiator, a curable composition preparation kit, a curable composition, a cured product, and a dental material that are obtained by using this.

### Solution to Problem

The specific means to solve the above problem include the following aspects.
<1> An ascorbic acid derivative or a salt thereof, comprising a structure in which at least one of hydroxyl groups included in an ascorbic acid is substituted by an ether bond bonded to a carbon atom.
<2> The ascorbic acid derivative or a salt thereof according to <1>, wherein the ascorbic acid derivative includes a structure represented by the following general formula (A). 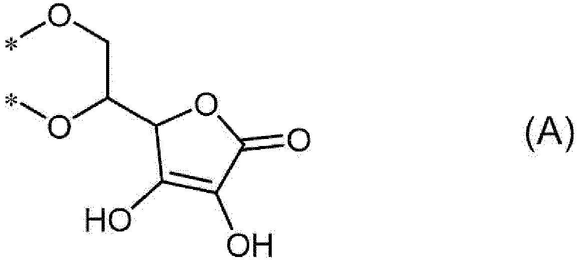 In the general formula (A), * is a bonding position with a carbon atom.
<3> The ascorbic acid derivative or a salt thereof according to <1> or <2>, wherein the ascorbic acid derivative contains a compound represented by the following general formula (B). 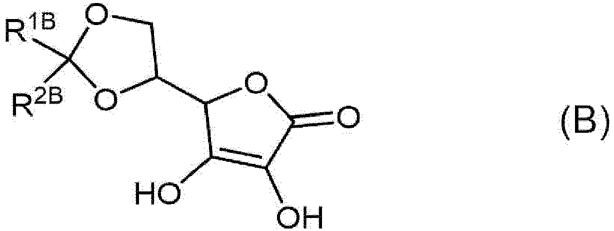 In the general formula (B), each of R^{1B} and R^{2B} independently represents a hydrogen atom or a monovalent organic group.
<4> The ascorbic acid derivative or a salt thereof according to <3>, wherein one of R^{1B} or R^{2B} is a hydrogen atom, the other of R^{1B} or R^{2B} is a monovalent organic group having 1 to 15 carbon atoms in the general formula (B), and the ascorbic acid derivative is a calcium salt.
<5> An additive for polymerization initiation, comprising the ascorbic acid derivative or salt thereof according to any one of <1> to <4>.
<6> A polymerization initiator comprising the ascorbic acid derivative or salt thereof according to any one of <1> to <4>, a transition metal compound, and an organic peroxide.
<7> A curable composition preparation kit, comprising:
   a first agent containing a monomer (A), and
   a second agent containing a monomer (B),
   wherein each of the ascorbic acid derivative or salt thereof according to any one of <1> to <4>, a transition metal compound and an organic peroxide are independently contained in at least one of the first agent or the second agent.
<8> The curable composition preparation kit according to <7>, wherein:
   the first agent contains the transition metal compound, and
   the second agent contains the ascorbic acid derivative or salt thereof.
<9> The curable composition preparation kit according to <7> or <8>, wherein one of the monomer (A) contained in the first agent or the monomer (B) contained in the second agent contains an acidic group-containing monomer.
<10> The curable composition preparation kit according to <9>, wherein:
   the monomer (A) contained in the first agent contains an acidic group-containing monomer, and
   the second agent contains the ascorbic acid derivative or a salt thereof.
<11> The curable composition preparation kit according to any one of <7> to <10>, wherein at least one of the first agent or the second agent contains at least one polymerization accelerator (1) selected from the group consisting of a phosphonite compound, a phosphite compound, and a sulfite compound.
<12> The curable composition preparation kit according to any one of <7> to <11>, wherein the second agent contains the ascorbic acid derivative or salt thereof and the polymerization accelerator (1).
<13> The curable composition preparation kit according to any one of <7> to <12>, wherein the first agent and the second agent contain a filler.
<14> The curable composition preparation kit according to any one of <7> to <13>, wherein a total content of the ascorbic acid derivative or salt thereof contained in the first agent and the second agent is from 0.1% by mass to 5% by mass with respect to a total amount of a curable composition to be prepared.
<15> A curable composition, comprising the ascorbic acid derivative or salt thereof according to any one of <1> to <4>, a transition metal compound, an organic peroxide and a monomer.
<16> A cured product of the curable composition according to <15>.
<17> A dental material, comprising the cured product according to <16>.

### Advantageous Effects of Invention

According to one embodiment of the present disclosure, an ascorbic acid derivative or a salt thereof than can improve adhesive properties of an obtained cured product, and an additive for polymerization initiation, a polymerization initiator, a curable composition preparation kit, a curable composition, a cured product, and a dental material that are obtained by using this, can be provided.

### DESCRIPTION OF EMBODIMENTS

In the present disclosure, those numerical ranges that are expressed with "to" each denote a range that includes the numerical values stated before and after "to" as the minimum value and the maximum value, respectively.

In a set of numerical ranges that are stated stepwise in the present disclosure, the upper limit value or the lower limit value of a numerical range may be replaced with the upper limit value or the lower limit value of other numerical range. Further, in a numerical range stated in the present disclosure the upper limit value or the lower limit value of the numerical range may be replaced with a value indicated in Examples.

In the present disclosure, a combination of two or more preferred embodiments is a more preferred embodiment.

In the present disclosure, when there are plural kinds of substances that correspond to a component, the indicated content of the component means, unless otherwise specified, the total content of the plural kinds of substances.

In the present disclosure, "(meth)acrylic" means acrylic and methacrylic, and "(meth)acryloyl" means acryloyl and methacryloyl.

In the present disclosure, "A or B" means at least one of A or B, and may include both A and B (for example, a mixture of A and B).

### <<Ascorbic acid derivative or salt thereof>>

An ascorbic acid derivative or a salt thereof of the present disclosure includes a structure in which at least one of hydroxyl groups included in an ascorbic acid is substituted by an ether bond bonded to a carbon atom. By using the ascorbic acid derivative or salt thereof, adhesive properties of an obtained cured product can be improved.

The ascorbic acid derivative or salt thereof may be an additive (additive for polymerization initiator) used for a polymerization initiator, or may be used as a polymerization initiator by combining another component (for example, transition metal compound, organic peroxide or the like).

Examples of the salt of the ascorbic acid derivative include an alkali metal salt of the ascorbic acid derivative, an alkaline earth metal salt of the ascorbic acid derivative or the like, and more specifically, example thereof include a sodium salt, a potassium salt, a magnesium salt, and a calcium salt of the ascorbic acid derivative, or the like. Among these, the calcium salt is preferable from the viewpoint of balance between polymerizability and adhesiveness.

The ascorbic acid derivative or salt thereof may include a structure in which at least one of the four hydroxyl groups included in ascorbic acid is substituted with an ether bond bonded to a carbon atom. It is preferable that it includes a structure in which each of two of the four hydroxyl groups included in ascorbic acid is substituted with an ether bond bonded to a carbon atom.

The ascorbic acid derivative of the present disclosure preferably includes a structure represented by the following general formula (A).

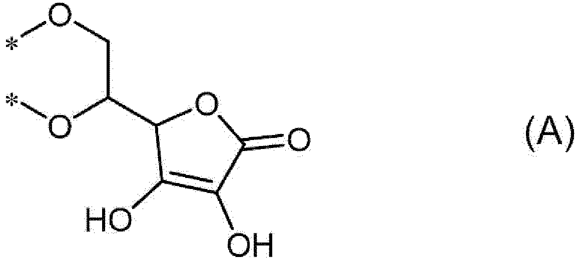

In the general formula (A), * represents the bonding position with a carbon atom. Two *'s in the general formula (A) may be bonded to the same carbon atom to form a 1,3-dioxolane skeleton.

The ascorbic acid derivative preferably includes a compound represented by the following general formula (B).

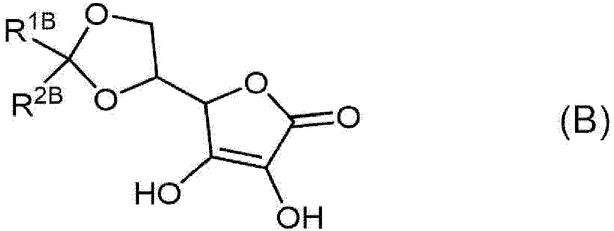

In the general formula (B), R^{1B}and R^{2B} are each independently a hydrogen atom or a monovalent organic group.

At least one of R^{1B} or R^{2B} is preferably a monovalent organic group.

The monovalent organic group in R^{1B} and R^{2B} is preferably an organic group having 1 to 12 carbon atoms, more preferably an organic group having 1 to 10 carbon atoms, and still more preferably an organic group having 3 to 10 carbon atoms.

On the other hand, from the balance between polymerizability and adhesive properties, the monovalent organic group in R^{1B} and R^{2B} is preferably an organic group having 1 to 15 carbon atoms, more preferably an organic group having 3 to 14 carbon atoms, and still more preferably an organic group having 8 to 12 carbon atoms.

In the general formula (B), one of R^{1B} or R^{2B} may be a hydrogen atom, and the other of R^{1B} or R^{2B} may be a monovalent organic group having 1 to 10 carbon atoms.

The monovalent organic group in R^{1B} and R^{2B} may be an organic group including an oxygen atom, a nitrogen atom, a sulfur atom, or the like, may be a hydrocarbon group to which an oxygen atom, a nitrogen atom, a sulfur atom, or the like is bonded, or may be a hydrocarbon group.

The salt of the ascorbic acid derivative may be a salt of a compound represented by the general formula (B), or may be an alkali metal salt or an alkaline earth metal salt of a compound represented by the general formula (B). From the viewpoint of balance between polymerizability and adhesive properties, a calcium salt of a compound represented by the general formula (B) is preferable.

When the salt of an ascorbic acid derivative is a salt of a compound represented by the general formula (B), each of R^{1B} and R^{2B} is independently preferably a hydrogen atom or a monovalent organic group. Furthermore, it is preferable that one of R^{1B} or R^{2B} is a hydrogen atom, and the other of R^{1B} or R^{2B} is a monovalent organic group having 1 to 10 carbon atoms, or each of R^{1B} and R^{2B} is independently a monovalent organic group having 1 to 10 carbon atoms.

When one of R^{1B} or R^{2B} is a hydrogen atom, and the other of R^{1B} or R^{2B} is a monovalent organic group having 1 to 10 carbon atoms, the other of R^{1B} or R^{2B} is preferably a monovalent organic group having 1 to 5 carbon atoms, more preferably a monovalent organic group having 1 to 3 carbon atoms, still more preferably a methyl group, ethyl group, propyl group or isopropyl group and particularly preferably an isopropyl group.

When each of R^{1B} and R^{2B} is independently a monovalent organic group having 1 to 10 carbon atoms, each of R^{1B} and R^{2B} is independently preferably a monovalent organic group having 1 to 5 carbon atoms, more preferably a monovalent organic group having 1 to 3 carbon atoms, still more preferably a methyl group, an ethyl group, a propyl group, or an isopropyl group and it is particularly preferable that both of them are methyl groups.

One example of a manufacturing method of an ascorbic acid derivative will be described below.

In this manufacturing method, ascorbic acid represented by the following general formula (C) is reacted with an aldehyde compound represented by the following general formula (D) or a ketone compound represented by the following general formula (E) by acetalization (see dashed line).

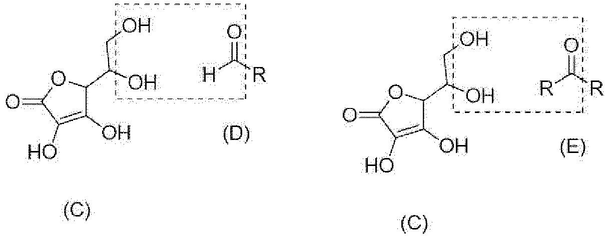

R in the above general formula (D) is the same as R^{1B}or R^{2B} in the aforementioned general formula (B).

Two R's in the above general formula (E) are the same as R^{1B}or R^{2B} in the aforementioned general formula (B). The two R's in the above general formula (E) may be the same or different.

Examples of the aldehyde compound represented by the above general formula (D) include butanal, isobutanal, hexanal, octanal, dodecanal, or the like. Examples of the ketone compound represented by the above general formula (E) include acetone, methyl ethyl ketone, methyl isobutyl ketone or the like.

These compounds may be used alone or in combination of two or more.

The lower limit of the reaction temperature in the acetalization reaction is not particularly limited and for example, is preferably 40°C or more, more preferably 50°C or more, and still more preferably 60°C or more.

The upper limit of the reaction temperature in the acetalization reaction is not particularly limited and for example, is preferably 90°C or less, more preferably 80°C or less, and still more preferably 70°C or less.

The lower limit of the reaction time in the acetalization reaction is not particularly limited and for example, is preferably 5 hours or more, more preferably 10 hours or more, and still more preferably 20 hours or more.

The upper limit of the reaction time in the acetalization reaction is not particularly limited and for example, is preferably 100 hours or less, more preferably 80 hours or less, and still more preferably 60 hours or less.

In the acetalization reaction, a solvent, an acetalization catalyst, or the like may be added, if necessary.

Examples of the solvent include amides such as dimethylacetamide and dimethylformamide. The solvent may be used alone or in combination of two or more.

The mixing ratio of the solvents is not particularly limited and is set appropriately depending on the purpose and application.

Examples of the acetalization catalyst include known acetalization catalysts such as an acid such as p-toluenesulfonic acid. The acetalization catalyst may be used alone or in combination of two or more.

The addition ratio of the esterification catalyst is set appropriately depending on the purpose and application.

Hereinafter, a method of manufacturing an alkali metal salt of an ascorbic acid derivative or an alkaline earth metal salt of an ascorbic acid derivative, which is the above salt of the ascorbic acid derivative will be explained.

In this method, an ascorbic acid represented by the following general formula (B) is reacted with an alkali metal salt or an alkaline earth metal salt represented by the following general formula (F).

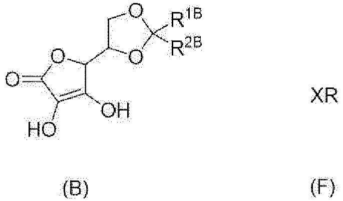

In the general formula (F), X represents an alkali metal or an alkaline earth metal, and R represents, for example, a carbonate group or a hydroxyl group.

Examples of the alkali metal salt or the alkaline earth metal represented by the above general formula (F) include sodium hydroxide, sodium hydrogen carbonate, sodium carbonate, calcium hydroxide, calcium carbonate or the like. These may be used alone or in combination of two or more.

The lower limit of the reaction temperature in the reaction with an alkali metal salt or an alkaline earth metal salt (hereinafter also referred to as "alkali metalation reaction") is not particularly limited and for example, is preferably 0°C or more, more preferably 10°C or more, and still more preferably 20°C or more.

The upper limit of the reaction temperature in the alkali metalation reaction is not particularly limited and for example, is preferably 100°C or less, more preferably 90°C or less, and still more preferably 80°C or less.

The lower limit of the reaction time in the alkali metalation reaction is not particularly limited and for example, is preferably 0.1 hours or more, more preferably 0.5 hours or more, and still more preferably 1 hour or more.

The upper limit of the reaction time in the alkali metalation reaction is not particularly limited and for example, is preferably 10 hours or less, more preferably 8 hours or less, and still more preferably 6 hour or less.

In the alkali metalation reaction, a solvent or the like may be added, if necessary.

Examples of the solvent include an alcohol such as methanol, ethanol, 2-propanol, butanol, or hexanol; a furan-type compound such as tetrahydrofuran; and a ketone-type compound such as acetone or methyl ethyl ketone, or the like. The solvent may be used alone or in combination of two or more.

The mixing ratio of the solvents is not particularly limited and is set appropriately depending on the purpose and application.

### <<Additive for polymerization initiation>>

An additive for polymerization initiation of the present disclosure contains the ascorbic acid derivative or salt thereof of the present disclosure. A combination of the additive for polymerization initiation of the present disclosure, and another component (for example, transition metal compound, organic peroxide or the like) can be used as a polymerization initiator.

The additive for polymerization initiation of the present disclosure may be an additive consisting only of the ascorbic acid derivative or salt thereof of the present disclosure, or may be an additive consisting of the ascorbic acid derivative or salt thereof of the present disclosure and another component.

### «Polymerization initiator»

The polymerization initiator according to the present disclosure contains an ascorbic acid derivative or a salt thereof according to the present disclosure, a transition metal compound, and an organic peroxide.

In the polymerization initiator according to the present disclosure, the total content of the ascorbic acid derivative and the salt thereof is preferably from 10 parts by mass to 70 parts by mass, more preferably from 15 parts by mass to 65 parts by mass, and still more preferably from 20 parts by mass to 60 parts by mass, with respect to 100 parts by mass of the polymerization initiator.

### <Transition metal compound>

The polymerization initiator according to the present disclosure contains a transition metal compound.

As the transition metal compound, a compound soluble in a monomer component in a curable composition preparation kit described later is preferable.

Examples of the transition metal compound include a copper compound, a vanadium compound, a molybdenum compound, a scandium compound, a titanium compound, a chromium compound, a manganese compound, an iron compound, a cobalt compound, a nickel compound, and the like.

Among the above, the transition metal compound preferably contains at least one of a copper compound or a vanadium compound, and more preferably contains a copper compound.

In the copper compound, examples of a copper carboxylate include copper acetate, copper isobutyrate, copper gluconate, copper citrate, copper phthalate, copper tartaric acid, copper oleate, copper octylate, copper octenate, copper naphthenate, copper methacrylate, and 4-cyclohexyl butyrate copper; examples of a β-diketone copper include acetylacetone copper, trifluoroacetyl acetone copper, hexafluoroacetylacetone copper, 2,2,6,6-tetramethyl-3,5-heptanedionato copper, and benzoylacetone copper; examples of a β-ketoester copper include ethyl acetoacetate copper; examples of a copper alkoxide include copper methoxide, copper ethoxide, copper isopropoxide, copper 2-(2-butoxyethoxy)ethoxide, and copper 2-(2-methoxyethoxy)ethoxide; examples of a copper dithiocarbamate include copper dimethyldithiocarbamate; examples of a salt of copper and inorganic acid include copper nitrate, and copper chloride.

These can be used alone or in combination of two or more as appropriate.

Among these, from the viewpoint of solubility and reactivity with respect to a monomer, a copper carboxylate, a β-diketone copper, and a β-ketoester copper is preferable, and copper acetate and acetylacetone copper are more preferable.

Examples of the vanadium compound include vandylacetylacetonate, vanadium naphthenate (III), vanadyl stearate, vanadium benzoylacetonate, bis(maltolato)oxovanadium (IV), oxobis(1-phenyl-1,3-butanedionate)vanadium (IV), and the like.

In the polymerization initiator according to the present disclosure, the content of the transition metal compound is preferably from 0.1 parts by mass to 1.5 parts by mass, more preferably from 0.2 parts by mass to 1 parts by mass, and still more preferably 0.3 parts by mass to 1 parts by mass, with respect to 100 parts by mass of the polymerization initiator.

### <Organic peroxide>

The polymerization initiator according to the present disclosure contains an organic peroxide.

Known organic peroxides can be used without a particular limitation. Examples of typical organic peroxides include hydroperoxide, peroxyester, ketone peroxide, peroxyketal, dialkyl peroxide, diacyl peroxide, peroxydicarbonate, and the like. Among these, hydroperoxide is preferable because even if the curable composition is provided in a packaged form and stored for a long period of time, there is little variation in the operable time. One type of organic peroxide may be used alone, or a plurality of types may be used in combination.

More specifically, examples of the hydroperoxide includes cumene hydroperoxide, t-butyl hydroperoxide, t-hexyl hydroperoxide, p-menthane hydroperoxide, diisopropylbenzene hydroperoxide, 1,1,3,3-tetramethylbutyl hydroperoxide, t-amyl hydroperoxide, or the like.

As the peroxy ester, any known peroxy ester may be used without any restriction as long as it includes an acyl group on one side of a peroxy group (-O-O- group) and a hydrocarbon group (or a similar group) on the other side. The specific examples include α,α-bis(neodecanoylperoxy)diisopropylbenzene, cumylperoxyneodecanoate, 1,1,3,3-tetramethylbutylperoxyneodecanoate, 1-cyclohexyl-1-methyl ethyl peroxy neodecanoate, t-hexyl peroxy neodecanoate, t-butyl peroxy neodecanoate, t-hexyl peroxy pivalate, t-butyl peroxy pivalate, 1,1,3,3-tetramethylbutylperoxy-2-ethylhexanoate, 2,5-dimethyl-2,5-bis(2-ethylhexanoylperoxy)hexane, 1-cyclohexyl-1-methylethylperoxy-2-ethylhexanoate, t-hexylperoxy 2-ethylhexanoate, t-butylperoxy 2-ethylhexanoate, t-butylperoxyisobutyrate, t-hexylperoxyisopropyl monocarbonate, t-butylperoxymaleic acid, t-butylperoxy 3,5,5-trimethylhexanoate, t-butylperoxylaurate, 2,5-dimethyl-2,5-bis(m-toluoylperoxy)hexane, t-butylperoxyisopropyl monocarbonate, t-butylperoxy 2-ethylhexyl monocarbonate, t-hexylperoxybenzoate, 2,5-dimethyl-2,5-bis(benzoylperoxy)hexane, t -butylperoxyacetate, t-butylperoxy-m-toluoylbenzoate, t-butylperoxybenzoate, bis(t-butylperoxy)isophthalate or the like. These can be used alone or in a combination of two or more as appropriate.

Examples of the ketone peroxide include methyl ethyl ketone peroxide, cyclohexanoperoxide, methylcyclohexanone peroxide, methyl acetoacetate peroxide, and acetylacetone peroxide or the like.

Examples of the peroxyketal include 1,1-bis(t-hexylperoxy)3,3,5-trimethylcyclohexane, 1,1-bis(t-hexylperoxy)cyclohexane, 1,1-bis(t-butyl peroxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, 2,2-bis(4,4-di-t-butylperoxycyclohexyl)propane, or the like.

Examples of the dialkyl peroxide include α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexane-3, or the like.

Examples of the diacyl peroxide include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, or a benzoyl peroxide-type compound.

Examples of the peroxydicarbonate include di-n-propyl peroxydicarbonate, diisopropyl peroxydicarbonate, bis(4-t-butylcyclohexyl) peroxydicarbonate, di-2-ethoxyethyl peroxydicarbonate, di-2-ethylhexyl peroxydicarbonate, di-2-methoxybutyl peroxydicarbonate, di(3-methyl-3-methoxybutyl)peroxydicarbonate, or the like.

In the polymerization initiator of the present disclosure, the content of the organic peroxide is preferably from 20 parts by mass to 90 parts by mass, more preferably from 30 parts by mass to 85 parts by mass, and still more preferably from 40 parts by mass to 80 parts by mass, with respect to 100 parts by mass of the polymerization initiator.

### «Curable composition preparation kit>>

A curable composition preparation kit of the present disclosure includes a first agent containing a monomer (A) and a second agent containing a monomer (B), and each of the ascorbic acid derivative or salt thereof of the present disclosure, a transition metal compound and an organic peroxide are independently contained in at least one of the first agent or the second agent.

From the viewpoint of suppressing the reaction between an ascorbic acid derivative or asalt thereof and a transition metal compound or an organic peroxide, it is preferable that the first agent contains the transition metal compound and the organic peroxide and the second agent contains the ascorbic acid derivative or salt thereof.

### <Monomer>

In the curable composition preparation kit of the present disclosure, the first agent contains a monomer (A), and the second agent contains a monomer (B).

The monomer (A) and monomer (B) may be the same monomer or may be different monomers.

As the monomer (A) and monomer (B), known monomers can be used.

The monomer (A) and monomer (B) may be a monomer that does not include an acidic group or may be a monomer that includes an acidic group (hereinafter also referred to as "acidic group-containing monomer").

It is preferable that the monomer (A) and monomer (B) contains a monomer that does not include an acidic group.

The monomer is a monomer that undergoes a radical polymerization reaction and becomes a polymer under the action of the ascorbic acid derivative or salt thereof of the present disclosure, a transition metal compound and an organic peroxide.

The monomer constituting the monomer in the present disclosure is not limited to one type, and two or more types may be used. Examples of the monomer that does not include an acidic group include a (meth)acrylate monomer that does not include an acidic group. Examples of the (meth)acrylate monomer that does not include an acidic group include a monofunctional monomer, a bifunctional monomer, or a trifunctional or higher functional monomer.

In the present disclosure, the content of the monomer (i.e., the total amount of the monomer (A) and monomer (B) in the curable composition to be prepared) is preferably from 10% by mass to 90% by mass, more preferably from 20% to 75% by mass, and still more preferably from 30% to 60% by mass, with respect to the total amount of the curable composition to be prepared.

Examples of the monofunctional monomer include 2-hydroxyethyl (meth)acrylate, 3-hydroxypropyl (meth)acrylate, 2-hydroxypropyl (meth)acrylate, 1,3-dihydroxypropyl (meth)acrylate, and 2,3-dihydroxypropyl (meth)acrylate. Among these, 2-hydroxyethyl methacrylate (HEMA) is preferable.

Examples of an aromatic compound-typed difunctional monomer include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxypolypropoxyphenyl)propane, or the like.

Among these, 2,2-bis[4-(3-(methacryloyloxy)-2-hydroxypropoxyphenyl)propane (commonly known as "Bis-GMA"), and 2,2-bis(4-methacryloyloxypolyethoxyphenyl)propane are preferable.

Examples of an aliphatic compound-typed difunctional monomer include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate , 1,10-decanediol di(meth)acrylate, 2,2,4-trimethylhexamethylenebis(2-carbamoyloxyethyl)dimethacrylate (UDMA), 1,2-bis(3-methacryloyloxy-2-hydroxypropyloxy)ethane or the like.

Among these, glycerol dimethacrylate, triethylene glycol dimethacrylate (TEGDMA), 1,6-hexanediol dimethacrylate (HexDMA), neopentyl glycol dimethacrylate (NPG), 2,2,4-trimethylhexamethylene bis(2-carbamoyloxyethyl)dimethacrylate (UDMA) and 1,2-bis(3-methacryloyloxy-2-hydroxypropyloxy)ethane are preferable.

Examples of the trifunctional or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacrylate, 1,7-diacryloyloxy-2,2,6,6-tetraacryloyloxymethyl-4-oxyheptane, or the like.

The above monomers may be blended singly or in combination of multiple types.

The amount of the above-mentioned monomer that does not include an acidic group is preferably in the range of 10 parts by mass to 100 parts by mass, more preferably in the range of 20 parts by mass to 100 parts by mass, and still more preferably in the range of 50 parts by mass to 100 parts by mass, with respect to 100 parts by mass of the total amount of monomer components in the curable composition preparation kit of the present disclosure.

In addition, when the monomer component in the curable composition preparation kit of the present disclosure includes an acidic group-containing monomer described below, the amount of the monomer that does not include an acidic group is preferably from 10 parts by mass to 99 parts by mass, more preferably from 30 parts by mass to 97 parts by mass, and still more preferably from 50 parts by mass to 95 parts by mass, with respect to 100 parts by mass of the total amount of monomer components in the curable composition preparation kit of the present disclosure.

The monomer (A) and monomer (B) preferably contain a (meth)acrylic monomer (C) having a molecular weight of 100 to 5,000.

The molecular weight of the (meth)acrylic monomer (C) is more preferably from 120 to 3,000, still more preferably from 150 to 2,000, and particularly preferably from 200 to 1,000.

The content of the (meth)acrylic monomer (C) with respect to the total content of the monomer (A) and monomer (B) is preferably 50% by mass or more, more preferably 70% by mass or more, and still more preferably 90% by mass or more.

In the curable composition preparation kit of the present disclosure, one of the monomer (A) contained in the first agent or the monomer (B) contained in the second agent preferably contains an acidic group-containing monomer.

Since at least one of the first agent or the second agent contains an acidic group-containing monomer, for example, when the curable composition preparation kit of the present disclosure is used for dental purposes, good tooth quality and high adhesion to a dental prosthetic material can be provided.

From the viewpoint of suppressing the reaction between the ascorbic acid derivative or salt thereof and an acidic group-containing monomer, it is preferable that the monomer (A) in the first agent contains the acidic group-containing monomer, and the second agent contains the ascorbic acid derivative or salt thereof, and it is more preferable that the monomer (A) in the first agent contains an acidic group-containing monomer, and the second agent does not include an acidic group-containing monomer and contains the ascorbic acid derivative or salt thereof.

Examples of the acidic group-containing monomer include a monomer that has at least one acidic group such as a phosphoric acid group, a pyrophosphoric acid group, a thiophosphoric acid group, a phosphonic acid group, a sulfonic acid group, or a carboxylic acid group, and that includes at least one polymerizable group such as an acryloyl group, a methacryloyl group, a vinyl group, or a styrene group.

The acidic group-containing monomer has an affinity with the adherend and has a demineralizing effect on the tooth substance.

Examples of a phosphate group-containing monomer include (meth)acryloyloxyalkyl dihydrogen phosphate such as 10-(meth)acryloyloxydecyl dihydrogen phosphate (MDP), bis[2-(meth)acryloyloxyethyl]hydrogen phosphate , bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9- (meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate and acid chlorides thereof, alkali metal salts thereof and ammonium salts thereof.

Examples of a pyrophosphate group-containing monomer include bis[2-(meth)acryloyloxyethyl] pyrophosphate, bis[4-(meth)acryloyloxybutyl] pyrophosphate, and acid chlorides thereof, alkali metal salts thereof, and ammonium salts thereof.

Examples of a thiophosphoric acid group-containing monomer include 2-(meth)acryloyloxyethyl dihydrogenthiophosphate, 3-(meth)acryloyloxypropyl dihydrogenthiophosphate, and acid chlorides thereof, alkali metal salts thereof, and ammonium salts thereof.

Examples of a phosphonic acid group-containing monomer include 2-(meth)acryloyloxyethyl phenylphosphonate, 5-(meth)acryloyloxypentyl-3-phosphonopropionate, and acid chlorides thereof, alkali metal salts thereof, and ammonium salts thereof.

Examples of a sulfonic acid group-containing monomer include 2-(meth)acrylamido-2-methylpropanesulfonic acid, styrenesulfonic acid, and 2-sulfoethyl (meth)acrylate.

Examples of a carboxylic acid group-containing monomer include a monomer containing one carboxy group in the molecule and a monomer containing multiple carboxy groups in the molecule.

Examples of the monomer containing one carboxy group in the molecule include (meth)acrylic acid, N-(meth)acryloylglycine, N-(meth)acryloyl aspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxybenzoic acid, ) acryloyloxyethyl hydrogen succinate, 2-(meth)acryloyloxyethyl hydrogen phthalate, 2-(meth)acryloyloxyethyl hydrogen malate, and acid halides thereof.

Examples of the monomer containing multiple carboxy groups in the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid. 11-(meth)acryloyloxyundecane-1,1-dicarboxylic acid, 12-(meth)acryloyloxydodecane-1,1-dicarboxylic acid, 13-(meth)acryloyloxy tridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, dihydroxyethyl methacrylate trimethylhexyldicarbamate, and acid anhydrides or acid halides thereof.

Among the above acidic group-containing monomers, from the point of high adhesive strength to an adherend, they are preferably 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2- methylpropanesulfonic acid, 11-(meth)acryloyloxyundecane-1,1-dicarboxylic acid, and dihydroxyethyl methacrylate trimethylhexyldicarbamate.

One type of the above acidic group-containing monomer may be used alone, or a plurality of types may be used in combination.

The amount of the acidic group-containing monomer is preferably from 1 parts by mass to 50 parts by mass, more preferably from 3 parts by mass to 40 parts by mass, and still more preferably from 5 parts by mass to 30 parts by mass, with respect to 100 parts by mass of the total amount of monomer components in the curable composition preparation kit of the present disclosure.

When the amount of the acidic group-containing monomer is 1 part by mass or more, it is easy to obtain high adhesiveness to various adherends.

Furthermore, when the amount of the acidic group-containing monomer is 50 parts by mass or less, it is easy to maintain a balance between polymerizability and adhesiveness. The total amount of monomer components means the total amount of the acidic group-containing monomer and the above-mentioned monomer that does not include an acidic group.

As for monomers in the present disclosure, for example, monomers described in known documents such as WO 2012/157566, WO 2015/015220, WO 2015/015221, and JP 2016-094482 can be used.

The total content of the ascorbic acid derivative or salt thereof contained in the first agent and the second agent is, from the viewpoint of adhesive properties, preferably from 0.1% by mass to 5% by mass, more preferably from 0.3% by mass to 3% by mass, and still more preferably from 0.5% by mass to 2% by mass, with respect to the total mass of the curable composition to be prepared.

The total content of the transition metal compound contained in the first agent and the second agent is, from the viewpoint of curability, preferably from 0.00005 parts by mass to 0.1 parts by mass, more preferably from 0.0001 parts by mass to 0.05 parts by mass, and still more preferably from 0.001 parts by mass to 0.03 parts by mass, with respect to 100 parts by mass of the total amount of monomer components in the curable composition preparation kit of the present disclosure.

The total content of the organic peroxide contained in the first agent and the second agent is, from the viewpoint of curability, preferably from 0.01 parts by mass to 6 parts by mass, more preferably from 0.01 parts by mass to 4 parts by mass, and still more preferably from 0.05 parts by mass to 3 parts by mass, with respect to 100 parts by mass of the total amount of monomer components in the curable composition preparation kit of the present disclosure.

In the curable composition preparation kit, at least one of the first agent or the second agent contains at least one polymerization accelerator (1) selected from the group consisting of a phosphonite compound, a phosphite compound, and a sulfite compound.

From the viewpoint that the polymerization accelerator (1) can function as a stabilizer for the ascorbic acid derivative or salt thereof, the second agent preferably contains the ascorbic acid derivative or salt thereof and the polymerization accelerator (1).

### <Phosphonite compound>

The phosphonite compound may be a trivalent organic phosphorus compound in which a carbon atom is bonded to a phosphorus atom. The phosphonite compound contained in the polymerization accelerator (1) of the present disclosure preferably includes a structure represented by the following general formula (I).

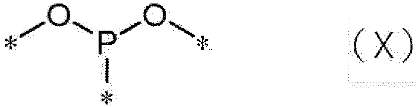

In the general formula (I), * is a bonding position with a carbon atom. Each of the three * is preferably a bonding position with a carbon atom included in a hydrocarbon group, and more preferably a bonding position with a carbon atom included in a benzene ring.

The phosphonite compound may include one structure represented by the general formula (I), or may include two or more structures represented by the general formula (I). The phosphonite compound preferably includes two structures represented by the general formula (I), and more preferably includes a skeleton in which two structures represented by the general formula (I) are bonded via a divalent linking group (preferably a biphenyl structure).

The phosphonite compound contained in the polymerization accelerator (1) of the present disclosure preferably contains a compound represented by the following general formula (II).

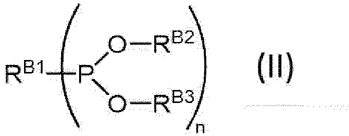

In the general formula (II), R^{B1} represents an n-valent hydrocarbon group, each of R^{B2} and R^{B3} independently represents a monovalent hydrocarbon group, and n is an integer 1 or 2.

Examples of the n-valent hydrocarbon group in R^{B1} include an n-valent aliphatic hydrocarbon group, an n-valent alicyclic hydrocarbon group, an n-valent aromatic hydrocarbon group, a combination of two or more of these, or the like.

A monovalent hydrocarbon group in R^{B1} is preferably an alkyl group, a phenyl group, a biphenyl group, or the like. A hydrogen atom of the phenyl group or biphenyl group in R^{B1} may be substituted with a substituent such as an alkyl group.

A divalent hydrocarbon group in R^{B1} is preferably a biphenylene group such as an alkylene group, a phenylene group, a 4,4'-biphenylene group, a 4,3'-biphenylene group, or a 3,3'-biphenylene group. A hydrogen atom included in the phenylene group or biphenylene group in R^{B1} may be substituted with a substituent such as an alkyl group.

Each of R^{B2} and R^{B3} independently represents a monovalent hydrocarbon group, and the monovalent hydrocarbon group is preferably an alkyl group, a phenyl group or the like. A hydrogen included in the phenyl group in R^{B2} and R^{B3} may be substituted with an alkyl group such as a tert-butyl group or n-butyl group.

n is an integer of 1 or 2, preferably 2.

Specific examples of the phosphonite compound contained in the polymerization accelerator (1) of the present disclosure include, for example, tetrakis(2,4-di-tert-butylphenyl)4,4'-biphenylene-di-phosphonite, tetrakis(2,4-di-tert-butyl-5-methylphenyl)4,4'-biphenylene-di-phosphonite, tetrakis(2,4-di-tert-butylphenyl)4,3'-biphenylene-di-phosphonite, tetrakis(2,4-di-tert-butylphenyl)3,3'-biphenylene-di-phosphonite, tetrakis(2,6-di-tert-butylphenyl)-4,4'-biphenylene-di-phosphonite, tetrakis(2,6- di-tert-butylphenyl)-4,3'-biphenylene-di-phosphonite, tetrakis(2,6-di-tert-butylphenyl)-3,3'-biphenylene-di-phosphonite, bis(2,4- di-tert-butylphenyl)-4-phenyl-phenylphosphonite, bis(2,4-di-tert-butylphenyl)-3-phenyl-phenylphosphonite, bis(2,6-di-n-butylphenyl)-3-phenyl-phenylphosphonite, bis(2,6-di-tert-butylphenyl)-4-phenyl-phenylphosphonite, bis(2,6-di-tert-butylphenyl)-3-phenyl-phenylphosphonite or the like.

Examples of the phosphite compound include triphenyl phosphite, trisnonylphenyl phosphite, tricresyl phosphite, diphenyl mono(2-ethylhexyl) phosphite, diphenyl monodecyl phosphite, diphenyl mono(tridecyl) phosphite, tris(2,4-di-tert-butylphenyl) phosphite, or the like.

Examples of the sulfite compound include ethylene sulfite, propylene sulfite, dimethyl sulfite, diethyl sulfite, ethyl methyl sulfite, methyl-n-propyl sulfite, ethyl-n-propyl sulfite, di-n-propyl sulfite, diphenyl sulfite, methylphenyl sulfite, ethyl sulfite, dibenzyl sulfite, benzylmethyl sulfite, benzylethyl sulfite, or the like.

The total content of the polymerization accelerator (1) contained in the first agent and the second agent is, from the viewpoint of curability, preferably from 0.1 parts by mass to 5 parts by mass and more preferably from 0.2 parts by mass to 2 parts by mass, with respect to 100 parts by mass of the total amount of the first agent and the second agent.

In the curable composition preparation kit of the present disclosure, at least one of the first agent or the second agent may contain a polymerization accelerator (2) other than the polymerization accelerator (1).

The polymerization accelerator (2) of the present disclosure is not particularly limited, examples thereof include an inorganic salt, thiourea or the like.

Examples of the inorganic salt include sodium sulfite, calcium sulfite, potassium sulfite, potassium nitrate, potassium chloride, potassium sulfate, sodium chloride or the like.

Examples of thiourea include acetylthiourea, phenylthiourea, triethylthiourea, tetramethylthiourea, dimethylthiourea, diphenylthiourea, or the like.

The total content of the polymerization accelerator (2) contained in the first agent and the second agent is, from the viewpoint of curability, preferably from 0.0001 parts by mass to 1 part by mass and more preferably from 0.001 parts by mass to 0.1 parts by mass, with respect to 100 parts by mass of the total amount of the first agent and the second agent.

The total content of the inorganic salt contained in the first agent and the second agent is, from the viewpoint of curability, preferably from 0.0001 parts by mass to 0.1 parts by mass and more preferably from 0.001 parts by mass to 0.01 parts by mass, with respect to 100 parts by mass of the total amount of the first agent and the second agent.

The total content of thiourea contained in the first agent and the second agent is, from the viewpoint of curability, preferably from 0.001 parts by mass to 1.0 part by mass and more preferably from 0.01 parts by mass to 0.1 parts by mass, with respect to 100 parts by mass of the total amount of the first agent and the second agent.

### <Filler>

In the curable composition preparation kit of the present disclosure, at least one of the first agent or the second agent may contain a filler, and it is preferable that the first agent and the second agent contain a filler.

One type of filler may be blended alone, or a combination of multiple types may be blended. Examples of the filler include an inorganic filler, an organic filler, and a composite filler of an inorganic filler and an organic filler.

Examples of the inorganic filler include silica; a mineral based on silica, such as kaolin, clay, mica, or mica; a ceramic or a glass which contains Al₂O₃, B₂O₃, TiO₂, ZrO₂, BaO, La₂O₃, SrO, ZnO, CaO, P₂O₅, Li₂O, Na₂O or the like.

As the glasses, lanthanum glass, barium glass, strontium glass, soda glass, lithium borosilicate glass, zinc glass, fluoroaluminosilicate glass, borosilicate glass, or bioglass is suitably used.

Crystalline quartz, hydroxyapatite, alumina, titanium oxide, yttrium oxide, zirconia, calcium phosphate, barium sulfate, aluminum hydroxide, sodium fluoride, potassium fluoride, sodium monofluorophosphate, lithium fluoride, or ytterbium fluoride is also preferably used.

Specifically, fine particle silica having a primary particle diameter of 0.001 µm to 0.1 µm is preferably used in terms of adhesive strength and ease of handling. Examples of commercially available products include "Aerosil OX50", "Aerosil 50", "Aerosil 200", "Aerosil 380", "Aerosil R972", and "Aerosil 130" (all manufactured by Nippon Aerosil Co., Ltd.).

Examples of the organic filler include polymethyl methacrylate, polyethyl methacrylate, a polyfunctional methacrylate polymer, polyamide, polystyrene, polyvinyl chloride, chloroprene rubber, nitrile rubber, or styrene-butadiene rubber.

Examples of the composite filler of the inorganic filler and the organic filler include a filler in which an inorganic filler is dispersed in an organic filler, and an inorganic/organic composite filler in which an inorganic filler is coated with a various polymer.

In order to improve curability, mechanical strength, and handleability, the filler may be used after being previously surface-treated with a known surface treatment agent such as a silane coupling agent. Examples of the surface treatment agent include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane or γ-aminopropyltriethoxysilane.

The total amount of the filler contained in the first agent and the second agent is preferably in the range of 10% by mass to 80% by mass, more preferably in the range of 30% by mass to 80% by mass, and still more preferably in the range of 50% by mass to 75% by mass, with respect to the total mass of the curable composition to be prepared.

### (Non-conductive filler)

In the curable composition preparation kit of the present disclosure, it is preferable that at least one of the first agent or the second agent contains a non-conductive filler.

A non-conductive filler means a filler with a resistance value of 1.00×10⁻⁴ Ωm or more.

The upper limit of the resistance value of the non-conductive filler is not particularly limited, and it may be, for example, 1.00×10²⁰ Ωm.

Examples of the material of the non-conductive filler include an organic material such as polyethylene, polystyrene, a phenol resin, an epoxy resin, an acrylic resin, or a benzoguanamine resin; silica (dimethylsilylated silica, or the like); a silicate (borosilicate glass (barium borosilicate glass, or the like)); an aluminosilicate glass (boroaluminosilicate glass, strontium boroaluminosilicate glass, fluoroaluminosilicate glass, barium aluminosilicate glass, or the like), a ceramic, an inorganic material such as boron nitride or barium nitride or the like.

Among the above materials, the materials of the non-conductive filler are preferably silica and silicate, and more preferably dimethylsilylated silica and barium aluminosilicate.

The first agent contains a non-conductive filler, and the content of the non-conductive filler with respect to the total mass of the first agent is preferably 10% by mass or more, more preferably 20% by mass or more, and still more preferably 30% by mass or more.

When the second agent contains a non-conductive filler, the second agent contains the non-conductive filler, and the content of the non-conductive filler with respect to the total mass of the second agent is preferably 10% by mass or more, more preferably 20% by mass or more, and still more preferably 30% by mass or more.

In the curable composition preparation kit of the present disclosure, when the non-conductive filler is contained in the first agent, the content of the non-conductive filler with respect to the total mass of the first agent is preferably 10% by mass or more or when the non-conductive filler is contained in the second agent, the content of the non-conductive filler with respect to the total mass of the second agent is preferably 10% by mass or more.

### <Additive>

In the curable composition preparation kit of the present disclosure, at least one of the first agent or the second agent may contain an additive such as a photopolymerization initiator, a stabilizer (polymerization inhibitor), a colorant, a fluorescent agent, an ultraviolet absorber or the like.

As the photopolymerization initiator, known photopolymerization initiators can be used, examples thereof include camphorquinone (CQ), ethyl dimethylaminobenzoate (EDB) or the like.

In addition, an antibacterial substance such as cetylpyridinium chloride, benzalkonium chloride, (meth)acryloyloxydodecylpyridinium bromide, (meth)acryloyloxyhexadecylpyridinium chloride, (meth)acryloyloxydecyl ammonium chloride, or triclosan may be blended.

A known dye or pigment may be blended into the curable composition of the present disclosure.

The curable composition preparation kit of the present disclosure is preferably used for a dental material.

The dental material is not particularly limited, and examples thereof include a dental adhesive, dental filling material, dental sealant (tooth fissure sealant), abutment building material, denture base resin, denture base lining material, dental crown prosthesis resin (hard resin for dental crown), dental room temperature polymerization resin, or the like.

The curable composition preparation kit of the present disclosure is particularly preferably used as a dental adhesive.

Examples of the dental adhesive include dental adhesive resin cement, orthodontic adhesive, adhesives for fixing loose teeth, cavity coating adhesive, and dental bonding material, or the like, and dental adhesive resin cement is preferable.

Examples of the dental filling material include a dental composite resin (including dental self-adhesive composite resin), root canal filling material, temporary sealant, backing material, or the like.

### <Curable Composition>

The curable composition of the present disclosure contains the ascorbic acid derivative or salt thereof of the present disclosure, a transition metal compound, an organic peroxide, and a monomer. When the curable composition of the present disclosure contains the above components, polymerizability can be favorably improved.

Specific examples, preferred embodiments, or the like of the monomer in the curable composition are the same as those for the above-mentioned monomer.

It is preferable that the curable composition of the present disclosure further contains a non-conductive filler.

Specific examples, preferred embodiments, or the like of the non-conductive filler in the curable composition are the same as those for the above-mentioned non-conductive filler.

The curable composition of the present disclosure may contain the aforementioned additive.

### <Cured Product>

The cured product of the present disclosure is a cured product of the curable composition of the present disclosure, or a cured product obtained using the curable composition preparation kit of the present disclosure.

The cured product of the present disclosure can be suitably used as a dental material. That is, the dental material of the present disclosure preferably includes the cured product of the present disclosure.

### EXAMPLES

Examples of the present disclosure will be shown below. This disclosure is not limited by the following examples.

Each component used in the examples is shown below.

### <Polymerization accelerator>

PPP: Tetrakis(2,4-di-tert-butylphenyl) [1,1'-biphenyl]-4,4'-diylbis(phosphonite)
NaCl: Sodium chloride
ATH: Acetylthiourea

### <Transition metal compound>

Cu(OAc)₂ monohydrate: copper acetate monohydrate

### <Filler>

GM27884: GM27884 manufactured by Schott, 1.5 µm, silane coupling agent processing amount 2.3%
R812: AEROSIL(R)R812 manufactured by Evonik Industries AG
SG-YBF100WSCMP10: Ytterbium filler, 0.1 µm

### <Ascorbic acid derivative with ether bond>

C4AAA: 3,4-dihydroxy-5-(2-propyl-1,3-dioxolan-4-yl)furan-2(5H)-one
C4isoAAA: 3,4-dihydroxy-5-(2-isopropyl-1,3-dioxolan-4-yl)furan-2(5H)-one
C6AAA: 3,4-dihydroxy-5-(2-pentyl-1,3-dioxolan-4-yl)furan-2(5H)-one
C12AAA: 3,4-dihydroxy-5-(2-undecyl-1,3-dioxolan-4-yl)furan-2(5H)-one
IPDAA: 5-(2,2-dimethyl-1,3-dioxolan-4-yl)-3,4-dihydroxyfuran-2(5H)-one

### <Salt of ascorbic acid derivative with ether bond >

C4AAANa: Sodium 4-hydroxy-5-oxo-2-(2-propyl-1,3-dioxolan-4-yl)-2,5-dihydroxyfuran-3-oleate
C4isoAAANa: Sodium 4-hydroxy-2-(2-propyl-1,3-dioxolan-4-yl)-5-oxo-2,5-dihydroxyfuran-3-oleate
C4isoAAACa: Calcium 4-hydroxy-2-(2-isopropyl-1,3-dioxolan-4-yl)-5-oxo-2,5-dihydroxyfuran-3-oleate
IPDAANa: Sodium 2-(2,2-dimethyl-1,3-dioxolan-4-yl)-4-hydroxy-5-oxo-2,5-dihydroxyfuran-3-oleate
IPDAACa: Calcium 2-(2,2-dimethyl-1,3-dioxolan-4-yl)-4-hydroxy-5-oxo-2,5-dihydroxyfuran-3-oleate
C6AAACa: Calcium 4-hydroxy-5-oxo-2-(2-pentyl-1,3-dioxolan-4-yl)-2,5-dihydroxyfuran-3-oleate
C12AAACa: Calcium 4-hydroxy-5-oxo-2-(2-undecyl-1,3-dioxolan-4-yl)-2,5-dihydroxyfuran-3-oleate

### <Another ascorbic acid-type compound>

AS6P: Ascorbic acid palmitoyl ester
AA: Ascorbic acid

### <Monomer that does not include acidic group>

2-HBMA: 2-hydroxybutyl methacrylate
TEGDMA: Triethylene glycol dimethacrylate
UDMA: Trimethylhexyl diurethane hydroxymethacrylate

### <Acidic group-containing monomer>

MDP: 10-methacryloyloxydecyl dihydrogen phosphate

### <Organic peroxide>

Luperox TAH: Tert-amyl hydroperoxide

### <Photopolymerization initiator>

BEDB: 2-butoxyethyl 4-(dimethylamino)benzoate
CQ: Camphorquinone

### <Polymerization inhibitor>

BHT: 2,6-di-tert-butyl-p-cresol

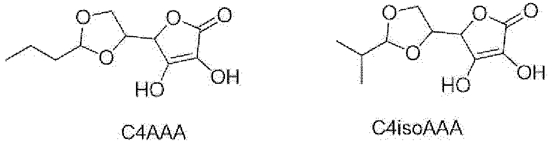

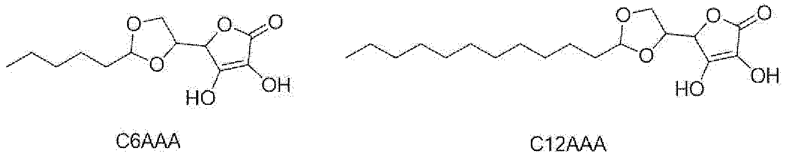

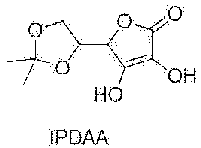

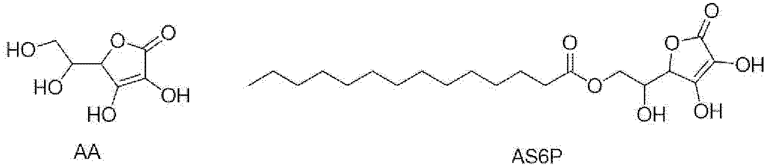

### <Synthesis Example 1 (C4AAA: Synthesis of 3,4-dihydroxy-5-(2-propyl-1,3-dioxolan-4-yl)furan-2(5H)-one)>

Ascorbic acid (10.0 g, 56.8 mmol) and dimethylacetamide (15 mL) as a solvent were charged into a device connected to a 200 mL eggplant-bottomed flask and a Dimroth condenser, and the temperature was raised to 60°C and the solution was stirred uniformly.

Then, n-butanal (4.04 g, 56.8 mmol) was added to the solution and the solution was stirred uniformly. Furthermore, p-toluenesulfonic acid monohydrate (1.14 g, 5.7 mmol), which is an acid catalyst, was added to the solution, and the solution was stirred uniformly. After 20 hours, the reaction was stopped and the mixture was cooled to room temperature.

Thereafter, the obtained reaction product was extracted. Specifically, an equal amount of distilled water was added to the reaction solution, then an equal amount of diethyl ether was added, and the mixture was extracted and washed using a separatory funnel. After repeating the above extraction three times, an equal amount of distilled water was added to the obtained organic layer and washed with a separatory funnel. After repeating the above washing three times, the resulting organic layer was dried by adding sodium sulfate, and then concentrated under reduced pressure. The obtained crude product was washed with a hexane/ethyl acetate solvent, and separated and purified by filtration.

Through the above steps, 3,4-dihydroxy-5-(2-propyl-1,3-dioxolan-4-yl)furan-2(5H)-one (C4AAA, 2.2 g, 9.56 mmol, yield 16.8%) was obtained. The attribution by ¹H-NMR(CD₃OD) of C4AAA is shown below.
¹H-NMR:50.91(t,3H,J=7.3Hz),1.39(td,2H,J=15.1Hz,7.5Hz),1.55(tt,2H,J=9.0Hz,2.6Hz),3.89-4.30(m,3H),4.67(dd,1H,J=12.6Hz,3.0Hz),4.86-4,90(m,1H)

### <Synthesis Example 2 (C4isoAAA: Synthesis of 3,4-dihydroxy-5-(2-isopropyl-1,3-dioxolan-4-yl)furan-2(5H)-one)>

Ascorbic acid (50.0 g, 284 mmol) and dimethylacetamide (105 mL) as a solvent were charged into a device connected to a 300 mL eggplant-bottomed flask and a Dimroth condenser, and the temperature was raised to 60°C and the solution was stirred uniformly.

Then, isobutanal (20.2 g, 284 mmol) was added to the solution and the solution was stirred uniformly. Furthermore, p-toluenesulfonic acid monohydrate (5.7 g, 28 mmol), which is an acid catalyst, was added to the solution, and the solution was stirred uniformly. After 20 hours, the reaction was stopped and the mixture was cooled to room temperature.

Thereafter, the obtained reaction product was extracted in the same manner as in Synthesis Example 1. 3,4-dihydroxy-5-(2-isopropyl-1,3-dioxolan-4-yl)furan-2(5H)-one (C4isoAAA, 16 g, 70 mmol, yield 24.6%) was obtained. The attribution by ¹H-NMR(CD₃OD) of C4isoAAA is shown below.
¹H-NMR:δ0.39(td,6H,J=4.SHz,2.2Hz),1.19-1.30(m,1H),3.39-3.79(m,3H),4.10-4.19(m,2H)

### <Synthesis Example 3 (C6AAA: Synthesis of 3,4-dihydroxy-5-(2-pentyl-1,3-dioxolan-4-yl)furan-2(5H)-one)>

Ascorbic acid (10.0 g, 56.4 mmol) and dimethylacetamide (15 mL) as a solvent were charged into a device connected to a 300 mL eggplant-bottomed flask and a Dimroth condenser, and the temperature was raised to 60°C and the solution was stirred uniformly.

Then, n-hexanal (5.69 g, 56.8 mmol) was added to the solution and the solution was stirred uniformly. Furthermore, p-toluenesulfonic acid monohydrate (1.14 g, 5.7 mmol), which is an acid catalyst, was added to the solution, and the solution was stirred uniformly. After 20 hours, the reaction was stopped and the mixture was cooled to room temperature.

Thereafter, the obtained reaction product was extracted in the same manner as in Synthesis Example 1. 3,4-dihydroxy-5-(2-pentyl-1,3-dioxolan-4-yl)furan-2(5H)-one (C6AAA, 4.73 g, 18.3 mmol, yield 32.2%) was obtained. The attribution by ¹H-NMR(CD₃OD) of C6AAA is shown below.
¹H-NMR:δ0.40(m,3H),0.76-0.92(m,6H),1.09(ddd,2H,J=17.1Hz,8.5Hz,4.4Hz),3.40-3.50(m,4H),3.59(dd,1H,J=8.5Hz,4.6Hz)

### <Synthesis Example 4 (C12AAA: Synthesis of 3,4-dihydroxy-5-(2-undecyl-1,3-dioxolan-4-yl)furan-2(5H)-one)>

Ascorbic acid (13.8 g, 78 mmol) and dimethylacetamide (60 mL) as a solvent were charged into a device connected to a 300 mL eggplant-bottomed flask and a Dimroth condenser, and the temperature was raised to 60°C and the solution was stirred uniformly.

Then, n-dodecanal (14.4 g, 78 mmol) was added to the solution and the solution was stirred uniformly. Furthermore, p-toluenesulfonic acid monohydrate (1.2 g, 7 mmol), which is an acid catalyst, was added to the solution, and the solution was stirred uniformly. After 20 hours, the reaction was stopped and the mixture was cooled to room temperature.

Thereafter, the obtained reaction product was extracted in the same manner as in Synthesis Example 1. Through the above steps, 3,4-dihydroxy-5-(2-undecyl-1,3-dioxolan-4-yl)furan-2(5H)-one (C12AAA, 3.8 g, 11.1 mmol, yield 14.2%) was obtained. The attribution by ¹H-NMR(CD₃OD) of C12AAA is shown below.
¹H-NMR:δ0.90(t,3H,J=6.9Hz),1.21-1.41(m,10H),1.54-1.62(m,3H),3.90-4.31(m,3H),4.66(dd,1H,J=12.1Hz,3.3Hz),4.87-4.90(m,1H)

### <Synthesis Example 5 (C4AAANa: Synthesis of sodium 4-hydroxy-5-oxo-2-(2-propyl-1,3-dioxolan-4-yl)-2,5-dihydroxyfuran-3-oleate)>

3,4-dihydroxy-5-(2-propyl-1,3-dioxolan-4-yl)furan-2(5H)-one (0.92 g, 4 mmol) and methanol (5 mL) as a solvent were charged into a 500 mL eggplant-bottomed flask, and the solution was stirred uniformly at room temperature. Further, an aqueous solution of sodium hydrogen carbonate (4 mmol, 5 mL) was added and the mixture was stirred and reacted. After 30 minutes, the reaction was stopped and the mixture was concentrated under reduced pressure. The obtained crude product was washed with methanol, and separated and purified by filtration.

Through the above steps, sodium 4-hydroxy-5-oxo-2-(2-propyl-1,3-dioxolan-4-yl)-2,5-dihydroxyfuran-3-oleate (C4AAANa, 1.0 g, 4 mmol, yield 99% white solid) was obtained. The attribution by ¹H-NMR(D₂O) of C4AAANa is shown below.
¹H-NMR:δ0.77(td,3H,J=7.4Hz,2.OHz),1.18-1.30(m,2H),1.47-1.52(m,2H),3.70-4.38(m,4H),4.85-4.89(m,1H)

### <Synthesis Example 6 (C4isoAAANa: Synthesis of sodium 4-hydroxy-2-(2-propyl-1,3-dioxolan-4-yl)-5-oxo-2,5-dihydroxyfuran-3-oleate)>

3,4-dihydroxy-5-(2-isopropyl-1,3-dioxolan-4-yl)furan-2(5H)-one(0.92 g, 4 mmol) and methanol (5 mL) as a solvent were charged into a 500 mL eggplant-bottomed flask, and the solution was stirred uniformly at room temperature. Further, an aqueous solution of sodium hydrogen carbonate (4 mmol, 5 mL) was added and the mixture was stirred and reacted. After 30 minutes, the reaction was stopped and the mixture was concentrated under reduced pressure. The obtained crude product was washed with methanol, and separated and purified by filtration.

Through the above steps, sodium 4-hydroxy-2-(2-isopropyl-1,3-dioxolan-4-yl)-5-oxo-2,5-dihydroxyfuran-3-oleate (C4isoAAANa, 1.0 g, 4 mmol, yield 99% white solid) was obtained. The attribution by ¹H-NMR(D₂O) of C4isoAAANa is shown below.
¹H-NMR:δ0.24-0.30(m,6H),1.13-1.26(m,1H),3.36-3.90(m,4H),4.11-4.12(m,1H)

### <Synthesis Example 7(C4isoAAACa: Synthesis of calcium 4-hydroxy-2-(2-isopropyl-1,3-dioxolan-4-yl)-5-oxo-2,5-dihydroxyfuran-3-oleate)>

3,4-dihydroxy-5-(2-isopropyl-1,3-dioxolan-4-yl)furan-2(5H)-one(2.3 g, 10 mmol) and methanol (50 mL) as a solvent were charged into a 500 mL eggplant-bottomed flask, and the solution was stirred uniformly at room temperature. Further, a 20 mM aqueous solution of calcium hydroxide (5 mmol, 250 mL) was added and the mixture was stirred and reacted. After 30 minutes, the reaction was stopped and the mixture was concentrated under reduced pressure. The obtained crude product was washed with methanol, and separated and purified by filtration.

Through the above steps, calcium 4-hydroxy-2-(2-isopropyl-1,3-dioxolan-4-yl)-5-oxo-2,5-dihydroxyfuran-3-oleate (C4isoAAACa, 1.8 g, 7.2 mmol, yield 72.2% white solid) was obtained. The attribution by ¹H-NMR(D₂O) of C4isoAAACa is shown below.
¹H-NMR:δ0.72-0.77(m,6H),1.60-1.72(m,1H),3.85-4.35(m,4H),4.58-4.60(m,1H)

### <Synthesis Example 8 (IPDAANa: Synthesis of sodium 2-(2,2-dimethyl-1,3-dioxolan-4-yl)-4-hydroxy-5-oxo-2,5-dihydroxyfuran-3-oleate)>

5-(2,2-dimethyl-1,3-dioxolan-4-yl)-3,4-dihydroxyfuran-2(5H)-one (0.86 g, 4 mmol) and methanol (50 mL) as a solvent were charged into a 500 mL eggplant-bottomed flask, and the solution was stirred uniformly at room temperature. Further, an aqueous solution of sodium hydrogen carbonate (4 mmol, 5 mL) was added and the mixture was stirred and reacted. After 30 minutes, the reaction was stopped and the mixture was concentrated under reduced pressure. The obtained crude product was washed with methanol, and separated and purified by filtration.

Through the above steps, sodium 2-(2,2-dimethyl-1,3-dioxolan-4-yl)-4-hydroxy-5-oxo-2,5-dihydroxyfuran-3-oleate (IPDAANa, 0.95 g, 4 mmol, yield 99% white solid) was obtained. The attribution by ¹H-NMR(D₂O) of IPDAANa is shown below.
¹H-NMR:δ1.25(s,3H),1.27(s,3H),4.07(ddd,2H,J=58.1Hz,8.8Hz,6.5Hz),4.33-4.38(m,2H)

### <Synthesis Example 9 (IPDAACa: Synthesis of calcium 2-(2,2-dimethyl-1,3-dioxolan-4-yl)-4-hydroxy-5-oxo-2,5-dihydroxyfuran-3-oleate)>

5-(2,2-dimethyl-1,3-dioxolan-4-yl)-3,4-dihydroxyfuran-2(SH)-one (2.16 g, 10 mmol) and methanol (10 mL) as a solvent were charged into a 500 mL eggplant-bottomed flask, and the solution was stirred uniformly at room temperature. Further, a 20mM aqueous solution of calcium hydroxide (5 mmol, 250 mL) was added and the mixture was stirred and reacted. After 30 minutes, the reaction was stopped and the mixture was concentrated under reduced pressure. The obtained crude product was washed with methanol, and separated and purified by filtration.

Through the above steps, calcium 2-(2,2-dimethyl-1,3-dioxolan-4-yl)-4-hydroxy-5-oxo-2,5-dihydroxyfuran-3-oleate (IPDAACa, 2.35 g, 10 mmol, yield 99% white solid) was obtained. The attribution by ¹H-NMR(D₂O) of IPDAACa is shown below.
¹H-NMR:δ1.24(s,3H),1.26(s,3H),3.99(dd,1H,J=8.9Hz,5.7Hz),4.14(dd,1H,J=8.8Hz,7.1Hz),4.32(d,1H,J =2.7Hz),4.34-4.38(m,1H)

### <Synthesis Example 10 (C6AAACa: Synthesis of calcium 4-hydroxy-5-oxo-2-(2-pentyl-1,3-dioxolan-4-yl)-2,5-dihydroxyfuran-3-oleate)>

3,4-dihydroxy-5-(2-pentyl-1,3-dioxolan-4-yl)furan-2(5H)-one (C6AAA, 2.58 g, 10 mmol) and methanol (25 mL) as a solvent were charged into a 500 mL eggplant-bottomed flask, and the solution was stirred uniformly at room temperature. Further, a 20 mM aqueous solution of calcium hydroxide (5 mmol, 250 mL) was added and the mixture was stirred and reacted. After 180 minutes, the reaction was stopped and the mixture was concentrated under reduced pressure.

Through the above steps, calcium 4-hydroxy-5-oxo-2-(2-pentyl-1,3-dioxolan-4-yl)-5-oxo-2,5-dihydroxyfuran-3-oleate (C6AAACa, 1.66 g, 6 mmol, yield 60% white solid) was obtained.

### <Synthesis Example 11 (C12AAACa: Synthesis of calcium 4-hydroxy-5-oxo-2-(2-undecyl-1,3-dioxolan-4-yl)-2,5-dihydroxyfuran-3-oleate)>

3,4-dihydroxy-5-(2-undecyl-1,3-dioxolan-4-yl)furan-2(5H)-one (C12AAA, 3.42 g, 10 mmol) and methanol (25 mL) as a solvent were charged into a 500 mL eggplant-bottomed flask, and the solution was stirred uniformly at room temperature. Further, a 20 mM aqueous solution of calcium hydroxide (5 mmol, 250 mL) was added and the mixture was stirred and reacted. After 300 minutes, the reaction was stopped and the precipitated solid was filtered with filter paper, the obtained solid was washed three times with methanol (25 mL), and the obtained solid was dried under reduced pressure.

Through the above steps, calcium 4-hydroxy-5-oxo-2-(2-undecyl -1,3-dioxolan-4-yl)-2,5-dihydroxyfuran-3-oleate (C12AAACa, 2.17 g, 6 mmol, yield 60% white solid) was obtained.

### <Examples 1 to 14 and Comparative Examples 1 to 3>

As shown in Tables 1 to 4, the first agent and the second agent of Examples 1 to 14 and Comparative Examples 1 to 3 were prepared using each component. In Tables 1 to 4, the numbers in each component column mean the amount (parts by mass) of each component in the first agent or the second agent.

### <Evaluation of adhesive properties>

A bovine mandibular anterior tooth was root-cut and pulp-extracted, placed in a plastic cylindrical container with a diameter of 25 mm and a depth of 25 mm, and embedded in an acrylic resin. This will be used as a bovine tooth adherend. The bovine tooth adherend was polished with waterproof emery paper (P400) immediately before use to carve out the smooth surface of the bovine dentin. Adhesive properties were evaluated in accordance with ISO 16506 using the first agent and second agent of each Example and Comparative Example that were separately prepared as test specimens used for measuring adhesive strength.

Specifically, the excess water on the adherend surface of the bovine tooth adherend is wiped off with a Kimwipe, and a drop of water is dropped onto the adherend. Next, the first agent and second agent listed in Tables 1 to 4 mixed for 20 seconds were applied to the adherend surface of a cylindrical cured product (Vinus Diamond, manufactured by Kulzer, previously polished with water-resistant emery paper P180) of a previously prepared filler composition with an appropriate amount, and the obtained cured product was placed vertically on the bovine tooth. Thereafter, pressure was applied using a dedicated jig with a force of 5N.

After removing the excess of the used composition, it was placed in a dedicated jig for 40 minutes at 37°C, then the jig was removed and left to stand at 37°C for 60 minutes, and then placed in a constant temperature bath at 37°C for 24 hours. Thereafter, the adhesive strength was measured.

The adhesive strength was determined by applying a shear load parallel to the bovine tooth adherend and in contact with the surface at a crosshead speed of 1.0 mm/min, and measuring the shear load at which the cylindrical cured product adhered to the bovine tooth adherend peeled off from the surface.

The adhesive properties of Examples 1 to 12 and Comparative Examples 1 to 3 were evaluated according to the following criteria 1, and those of Examples 13 and 14 were evaluated according to the following criteria 2, which is a further subdivision of A and B in criteria 1.

Criteria 1 is evaluation criteria for Examples and Comparative Examples in which the content of the ascorbic acid-type compound was kept under the same conditions.

Criteria 2 is evaluation criteria for Examples in which the number of moles of the ascorbic acid-type compound was set to the same conditions.

### (Criteria 1)

A: Adhesive strength is 15 MPa or more and less than 23 MPa.
B: Adhesive strength is 13 MPa or more and less than 15 MPa.
C: Adhesive strength is 10 MPa or more and less than 13 MPa.
D: Adhesive strength is less than 10 MPa.
E: All adherends were detached before the test, making it impossible to evaluate adhesive properties.

### (Criteria 2)

A1: Adhesive strength is 17 MPa or more and less than 23 MPa.
A2: Adhesive strength is 15 MPa or more and less than 17 MPa.
B1: Adhesive strength is 14 MPa or more and less than 15 MPa.
B2: Adhesive strength is 13 MPa or more and less than 14 MPa.

**[Table1]**

| | | Example | | | | | | | | Comparative Example | | | | | |
|---|---|---|---|---|---|---|---|---|---|---|---|---|---|---|---|
| | | 1 | | 2 | | 3 | | 4 | | 1 | | 2 | | 3 | |
| | | First agent | Second agent | First agent | Second agent | First agent | Second agent | First agent | Second agent | First agent | Second agent | First agent | Second agent | First agent | Second agent |
| Polymerization accelerator | PPP | | 0.500 | | 0.500 | | 0.500 | | 0.500 | | 0.500 | | 0.500 | | 0.500 |
| | NaCl | | 0.015 | | 0.015 | | 0.015 | | 0.015 | | 0.015 | | 0.015 | | 0.015 |
| Transition metal compound | Cu(OAc)₂ monohydrate | 0.030 | | 0.030 | | 0.030 | | 0.030 | | 0.030 | | 0.030 | | 0.030 | |
| Filler | GM27884 | 56.304 | 65.067 | 56.304 | 65.067 | 56.304 | 65.067 | 56.304 | 65.067 | 56.304 | 65.067 | 56.304 | 65.067 | 56.304 | 66.066 |
| | R812 | 1.300 | 1.481 | 1.300 | 1.481 | 1.300 | 1.481 | 1.300 | 1.481 | 1.300 | 1.481 | 1.300 | 1.481 | 1.300 | 1.504 |
| Ascorbic acid derivative with ether bond | C4 AAA | | 1.500 | | | | | | | | | | | | |
| | C4iso AAA | | | | 1.500 | | | | | | | | | | |
| | C6 AAA | | | | | | 1.500 | | | | | | | | |
| | C12AAA | | | | | | | | 1.500 | | | | | | |
| Another ascorbic acid-type compound | AS6P | | | | | | | | | | 1.500 | | | | |
| | As | | | | | | | | | | | | 1.500 | | |
| Monomer that does not include acidic group | 2-HBMA | 1.732 | 1.974 | 1.732 | 1.974 | 1.732 | 1.974 | 1.732 | 1.974 | 1.732 | 1.974 | 1.732 | 1.974 | 1.732 | 2.005 |
| | TEGDMA | 7.796 | 8.885 | 7.796 | 8.885 | 7.796 | 8.885 | 7.796 | 8.885 | 7.796 | 8.885 | 7.796 | 8.885 | 7.796 | 9.023 |
| | UDMA | 18.794 | 20.328 | 18.794 | 20.328 | 18.794 | 20.328 | 18.794 | 20.328 | 18.794 | 20.328 | 18.794 | 20.328 | 18.794 | 20.636 |
| Acidic group-containing monomer | MDP | 10.000 | | 10.000 | | 10.000 | | 10.000 | | 10.000 | | 10.000 | | 10.000 | |
| Organic peroxide | Luperox TAH | 4.000 | | 4.000 | | 4.000 | | 4.000 | | 4.000 | | 4.000 | | 4.000 | |
| Photopolymerization initiator | BEDB | | 0.100 | | 0.100 | | 0.100 | | 0.100 | | 0.100 | | 0.100 | | 0.100 |
| | CQ | | 0.100 | | 0.100 | | 0.100 | | 0.100 | | 0.100 | | 0.100 | | 0.100 |
| Polymerization inhibitor | BHT | 0.044 | 0.050 | 0.044 | 0.050 | 0.044 | 0.050 | 0.044 | 0.050 | 0.044 | 0.050 | 0.044 | 0.050 | 0.044 | 0.051 |
| Total | | 100.000 | 100.000 | 100.000 | 100.000 | 100.000 | 100.000 | 100.000 | 100.000 | 100.000 | 100.000 | 100.000 | 100.000 | 100.000 | 100.000 |
| Adhesive properties | A | A | A | A | D | C | E | | | | | | | | |

**[Table 2]**

| | | Example | | | | | | | | | |
|---|---|---|---|---|---|---|---|---|---|---|---|
| | | 5 | | 6 | | 7 | | 8 | | 9 | |
| | | First agent | Second agent | First agent | Second agent | First agent | Second agent | First agent | Second agent | First agent | Second agent |
| Polymerization accelerator | PPP | | 0.500 | | 0.500 | | 0.500 | | 0.500 | | 0.500 |
| | NaCl | | 0.015 | | 0.015 | | 0.015 | | 0.015 | | 0.015 |
| Transition metal compound | Cu(OAc)₂ monohydrate | 0.030 | | 0.030 | | 0.030 | | 0.030 | | 0.030 | |
| Filler | GM27884 UF1.5 Sil2.3% | 56.304 | 0.900 | 56.304 | 0.900 | 56.304 | 0.900 | 56.304 | 0.900 | 56.304 | 0.900 |
| | R812 | 1.300 | 1.481 | 1.300 | 1.481 | 1.300 | 1.481 | 1.300 | 1.481 | 1.300 | 1.481 |
| | SG-YBF100WSCMP10 | | 64.167 | | 64.167 | | 64.167 | | 64.167 | | 64.167 |
| Salt of ascorbic acid with ether bond | C4isoAAANa | | 1.500 | | | | | | | | |
| | C4isoAAACa | | | | 1.500 | | | | | | |
| | C4AAANa | | | | | | 1.500 | | | | |
| | IPDAANa | | | | | | | | 1.500 | | |
| | IPDAACa | | | | | | | | | | 1.500 |
| Monomer that does not include acidic group | 2-HBMA | 1.732 | 1.974 | 1.732 | 1.974 | 1.732 | 1.974 | 1.732 | 1.974 | 1.732 | 1.974 |
| | TEGDMA | 7.796 | 8.885 | 7.796 | 8.885 | 7.796 | 8.885 | 7.796 | 8.885 | 7.796 | 8.885 |
| | UDMA | 18.794 | 20.328 | 18.794 | 20.328 | 18.794 | 20.328 | 18.794 | 20.328 | 18.794 | 20.328 |
| Acidic group-containing monomer | MDP | 10.000 | | 10.000 | | 10.000 | | 10.000 | | 10.000 | |
| Organic peroxide | Luperox TAH | 4.000 | | 4.000 | | 4.000 | | 4.000 | | 4.000 | |
| Photopolymerization initiator | BEDB | | 0.100 | | 0.100 | | 0.100 | | 0.100 | | 0.100 |
| | CQ | | 0.100 | | 0.100 | | 0.100 | | 0.100 | | 0.100 |
| Polymerization inhibitor | BHT | 0.044 | 0.050 | 0.044 | 0.050 | 0.044 | 0.050 | 0.044 | 0.050 | 0.044 | 0.050 |
| Total | | 100.000 | 100.000 | 100.000 | 100.000 | 100.000 | 100.000 | 100.000 | 100.000 | 100.000 | 100.000 |
| Adhesive properties | A | A | A | A | A | | | | | | |

**[Table 3]**

| | | Example | | | | | |
|---|---|---|---|---|---|---|---|
| | | 10 | | 11 | | 12 | |
| | | First agent | Second agent | First agent | Second agent | First agent | Second agent |
| Polymerization accelerator | ATH | | 0.150 | | 0.150 | | 0.150 |
| | PPP | | 1.000 | | 1.000 | | 1.000 |
| Transition metal compound | Cu(OAc)₂ monohydrate | 0.020 | | 0.020 | | 0.020 | |
| Filler | GM27884 UF1.5 Sil2.3% | 57.304 | | 57.304 | | 57.304 | |
| | R812 | 1.323 | 1.494 | 1.323 | 1.494 | 1.323 | 1.494 |
| | SG-YBF100WSCMP10 | | 64.733 | | 64.733 | | 64.733 |
| Ascorbic acid derivative | IPDAACa | | 1.500 | | | | |
| | C4isoAAACa | | | | 1.500 | | |
| | C12AAACa | | | | | | 1.500 |
| Monomer that does not include acidic group | 2-HBMA | 1.763 | 1.992 | 1.763 | 1.992 | 1.763 | 1.992 |
| | TEGDMA | 7.934 | 8.963 | 7.934 | 8.963 | 7.934 | 8.963 |
| | UDMA | 19.612 | 19.918 | 19.612 | 19.918 | 19.612 | 19.918 |
| Acidic group-containing monomer | MDP | 10.000 | | 10.000 | | 10.000 | |
| Organic peroxide | Luperox TAH | 2.000 | | 2.000 | | 2.000 | |
| Photopolymerization initiator | BEDB | | 0.100 | | 0.100 | | 0.100 |
| | CQ | | 0.100 | | 0.100 | | 0.100 |
| Polymerization inhibitor | BHT | 0.044 | 0.050 | 0.044 | 0.050 | 0.044 | 0.050 |
| Total | | 100.000 | 100.000 | 100.000 | 100.000 | 100.000 | 100.000 |
| Adhesive properties | A | A | A | | | | |

**[Table 4]**

| | | Molucular weight | Example | | | |
|---|---|---|---|---|---|---|
| | | | 13 | | 14 | |
| | | | First agent | Second agent | First agent | Second agent |
| Polymerization accelerator | ATH | | | 0.150 | | 0.150 |
| | PPP | | | 1.000 | | 1.000 |
| Transition metal compound | Cu(OAc)₂ monohydrate | | 0.020 | | 0.020 | |
| Filler | GM27884 UF1.5 Sil2.3% | | 57.304 | | 57.304 | |
| | R812 | | 1.323 | 1.494 | 1.323 | 1.494 |
| | SG-YBF100WSCMP10 | | | 64.733 | | 64.733 |
| Ascorbic acid derivative | C6AAACa | 554.60 | | 1.768 | | |
| | C12AAACa | 722.93 | | | | 2.305 |
| Monomer that does not include acidic group | 2-HBMA | | 1.763 | 1.980 | 1.763 | 1.980 |
| | TEGDMA | | 7.934 | 8.950 | 7.934 | 8.588 |
| | UDMA | | 19.612 | 19.675 | 19.612 | 19.500 |
| Acidic group-containing monomer | MDP | | 10.000 | | 10.000 | |
| Organic peroxide | Luperox TAH | | 2.000 | | 2.000 | |
| Photopolymerization initiator | BEDB | | | 0.100 | | 0.100 |
| | CQ | | | 0.100 | | 0.100 |
| Polymerization inhibitor | BHT | | 0.044 | 0.050 | 0.044 | 0.050 |
| Total | | | 100.000 | 100.000 | 100.000 | 100.000 |
| Adhesive properties | | B2 | A1 | | | |

As shown in Tables 1 to 3, in Examples 1 to 12, each of which uses the specific ascorbic acid derivative or salt thereof, the adhesive properties of the cured products could be improved better than in Comparative Examples 1 and 2.

As shown in Table 4, when the adhesive properties of the cured products of Examples 13 and 14 were compared after the molar number of the calcium salt of the ascorbic acid derivative was made the same, the adhesive properties of Example 14 (using C12AAACa as the calcium salt of the ascorbic acid derivative) were superior to those of Example 13 (using C6AAACa as the calcium salt of the ascorbic acid derivative).

The entire contents of the disclosures by Japanese Patent Application No. 2022-050804 filed on March 25, 2022, are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

## Claims

1. An ascorbic acid derivative or a salt thereof, comprising a structure in which at least one of hydroxyl groups included in an ascorbic acid is substituted by an ether bond bonded to a carbon atom.

2. The ascorbic acid derivative or a salt thereof according to claim 1, wherein the ascorbic acid derivative includes a structure represented by the following general formula (A): 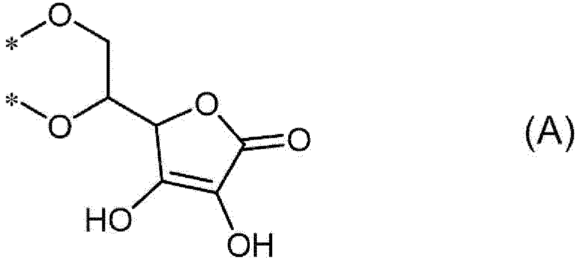 wherein, in the general formula (A), * is a bonding position with a carbon atom.

3. The ascorbic acid derivative or a salt thereof according to claim 1 or 2, wherein the ascorbic acid derivative contains a compound represented by the following general formula (B): 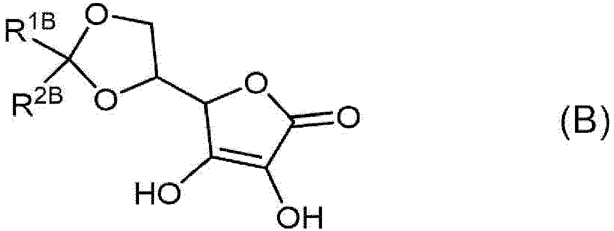 wherein, in the general formula (B), each of R^{1B} and R^{2B} independently represents a hydrogen atom or a monovalent organic group.

4. The ascorbic acid derivative or a salt thereof according to claim 3, wherein one of R^{1B} or R^{2B} is a hydrogen atom, the other of R^{1B} or R^{2B} is a monovalent organic group having 1 to 15 carbon atoms in the general formula (B), and the ascorbic acid derivative is a cm salt.

5. An additive for polymerization initiation, comprising the ascorbic acid derivative or salt thereof according to any one of claims 1 to 4.

6. A polymerization initiator comprising the ascorbic acid derivative or salt thereof according to any one of claims 1 to 4, a transition metal compound, and an organic peroxide.

7. A curable composition preparation kit, comprising:
a first agent containing a monomer (A), and
a second agent containing a monomer (B),
wherein each of the ascorbic acid derivative or salt thereof according to any one of claims 1 to 4, a transition metal compound and an organic peroxide are independently contained in at least one of the first agent or the second agent.

8. The curable composition preparation kit according to claim 7, wherein:
the first agent contains the transition metal compound, and
the second agent contains the ascorbic acid derivative or salt thereof.

9. The curable composition preparation kit according to claim 7 or 8, wherein one of the monomer (A) contained in the first agent or the monomer (B) contained in the second agent contains an acidic group-containing monomer.

10. The curable composition preparation kit according to claim 9, wherein:
the monomer (A) contained in the first agent contains an acidic group-containing monomer, and
the second agent contains the ascorbic acid derivative or a salt thereof.

11. The curable composition preparation kit according to any one of claims 7 to 10, wherein at least one of the first agent or the second agent contains at least one polymerization accelerator (1) selected from the group consisting of a phosphonite compound, a phosphite compound, and a sulfite compound.

12. The curable composition preparation kit according to any one of claims 7 to 11, wherein the second agent contains the ascorbic acid derivative or salt thereof and the polymerization accelerator (1).

13. The curable composition preparation kit according to any one of claims 7 to 12, wherein the first agent and the second agent contain a filler.

14. The curable composition preparation kit according to any one of claims 7 to 13, wherein a total content of the ascorbic acid derivative or salt thereof contained in the first agent and the second agent is from 0.1% by mass to 5% by mass with respect to a total amount of a curable composition to be prepared.

15. A curable composition, comprising the ascorbic acid derivative or salt thereof according to any one of claims 1 to 4, a transition metal compound, an organic peroxide and a monomer.

16. A cured product of the curable composition according to claim 15.

17. A dental material, comprising the cured product according to claim 16.
